(19) Europäisches Patentamt / European Patent Office / Office européen des brevets

(11) **EP 2 821 069 A1**

(12) # EUROPEAN PATENT APPLICATION
published in accordance with Art. 153(4) EPC

(43) Date of publication:
**07.01.2015 Bulletin 2015/02**

(21) Application number: **13755366.5**

(22) Date of filing: **01.03.2013**

(51) Int Cl.:
*A61K 31/198* [(2006.01)]  *A61P 1/00* [(2006.01)]
*A61P 1/10* [(2006.01)]  *A61P 1/14* [(2006.01)]
*A23L 1/305* [(2006.01)]  *A23L 2/52* [(2006.01)]

(86) International application number:
**PCT/JP2013/055617**

(87) International publication number:
**WO 2013/129642 (06.09.2013 Gazette 2013/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **02.03.2012 JP 2012046486**

(71) Applicant: **Kyowa Hakko Bio Co., Ltd.
Tokyo 100-8185 (JP)**

(72) Inventors:
• **OHINATA, Kousaku
Uji-shi
Kyoto 611-0011 (JP)**

• **HO, Yee, Yin
Uji-shi
Kyoto 611-0011 (JP)**
• **MIZUSHIGE, Takafumi
Uji-shi
Kyoto 611-0011 (JP)**
• **KANEKO, Kentaro
Uji-shi
Kyoto 611-0011 (JP)**
• **AKIZUKI, Saori
Tsukuba-shi
Ibaraki 305-0841 (JP)**
• **MORISHITA, Koji
Tokyo 100-8185 (JP)**

(74) Representative: **Vossius & Partner
Siebertstrasse 4
81675 München (DE)**

(54) **ENHANCER FOR EATING ACTIVITY AND/OR GASTROINTESTINAL ACTIVITY**

(57) An agent for secreting ghrelin, comprising ornithine or a salt thereof as an active ingredient, an agent for enhancing eating activity, comprising ornithine or a salt thereof as an active ingredient, and an agent for enhancing gastrointestinal activity, comprising ornithine or a salt thereof as an active ingredient.

EP 2 821 069 A1

**Description**

Technical Field

The present invention relates to an agent for enhancing eating activity and/or gastrointestinal activity comprising ornithine or a salt thereof as an active ingredient.

Background Art

L-Ornithine has been used as a food material for enhancing muscle synthesis, or increasing basal metabolism to prevent obesity, primarily in the United States. In Europe, L-ornithine is used in the form of L-ornithine L-aspartate as a pharmaceutical agent for ameliorating liver disorders.
Other known effects of ornithine include amelioration of subjective symptom of fatigue (Patent Document 1), amelioration of sleeping or waking (Patent Document 2), amelioration of skin condition (Patent Document 3), amelioration of sensitivity to cold (Patent Document 4), and promotion of collagen synthesis (Patent Document 5).
However, it has not been known that ingestion of ornithine or a salt thereof enhances eating activity and gastrointestinal activity.

Prior Art

Patent Document

Patent Document 1: WO2007/040244
Patent Document 2: JP-A-2006-342148
Patent Document 3: JP-A-200 7-031375
Patent Document 4: JP-A-2007-119348
Patent Document 5: JP-A-2008-214232

Summary of Invention

Problems to be Solved by the Invention

An object of the present invention is to provide an agent for preventing or ameliorating reduced eating activity or reduced gastrointestinal activity; a symptom associated with impairment in eating which is caused by the progress of reduced eating activity, and which does not involve organic injury in the gastrointestinal tract (impairment in eating due to functional dyspepsia, impairment in eating due to age-related gastrointestinal malfunction, or the like); a symptom caused by the progress of reduced gastrointestinal activity (indigestion, constipation, cachexia, anorexia nervosa, functional dyspepsia, or wasting disease); or the like.
The present invention relates to the following (1) to (32).

(1) An agent for secreting ghrelin, comprising ornithine or a salt thereof as an active ingredient.
(2) An agent for enhancing eating activity, comprising ornithine or a salt thereof as an active ingredient.
(3) An agent for preventing or ameliorating a symptom caused by reduced eating activity, comprising ornithine or a salt thereof as an active ingredient.
(4) The agent for prevention or amelioration according to (3), wherein the symptom caused by reduced eating activity is impairment in eating which does not involve organic injury in the gastrointestinal tract.
(5) The agent for prevention or amelioration according to (4), wherein the impairment in eating which does not involve organic injury in the gastrointestinal tract is at least one symptom selected from impairment in eating due to functional dyspepsia, and impairment in eating due to age-related gastrointestinal malfunction.
(6) An agent for enhancing gastrointestinal activity, comprising ornithine or a salt thereof as an active ingredient.
(7) An agent for preventing or ameliorating a symptom caused by reduced gastrointestinal activity, comprising ornithine or a salt thereof as an active ingredient.
(8) The agent for prevention or amelioration according to (7), wherein the symptom caused by reduced gastrointestinal activity is at least one symptom selected from indigestion, constipation, cachexia, anorexia nervosa, functional dyspepsia, and wasting disease.
(9) A method for secreting ghrelin comprising the step of administering an effective amount of ornithine or a salt thereof.

(10) A method for enhancing eating activity comprising the step of administering an effective amount of ornithine or a salt thereof.

(11) A method for preventing or ameliorating a symptom caused by reduced eating activity, comprising the step of administering an effective amount of ornithine or a salt thereof.

(12) The method for prevention or amelioration according to (11), wherein the symptom caused by reduced eating activity is impairment in eating which does not involve organic injury in the gastrointestinal tract.

(13) The method for prevention or amelioration according to (12), wherein the impairment in eating which does not involve organic injury in the gastrointestinal tract is at least one symptom selected from impairment in eating due to functional dyspepsia, and impairment in eating due to age-related gastrointestinal malfunction.

(14) A method for enhancing gastrointestinal activity, comprising the step of administering an effective amount of ornithine or a salt thereof.

(15) A method for preventing or ameliorating a symptom caused by reduced gastrointestinal activity, comprising the step of administering an effective amount of ornithine or a salt thereof.

(16) The method for prevention or amelioration according to (15), wherein the symptom caused by reduced gastrointestinal activity is at least one symptom selected from indigestion, constipation, cachexia, anorexia nervosa, functional dyspepsia, and wasting disease.

(17) Use of ornithine or a salt thereof for the manufacture of an agent for secreting ghrelin.

(18) Use of ornithine or a salt thereof for the manufacture of an agent for enhancing eating activity.

(19) Use of ornithine or a salt thereof for the manufacture of an agent for preventing or ameliorating a symptom caused by reduced eating activity.

(20) The use according to (19), wherein the symptom caused by reduced eating activity is impairment in eating which does not involve organic injury in the gastrointestinal tract.

(21) The use according to (20), wherein the impairment in eating which does not involve organic injury in the gastrointestinal tract is at least one symptom selected from impairment in eating due to functional dyspepsia, and impairment in eating due to age-related gastrointestinal malfunction.

(22) Use of ornithine or a salt thereof for the manufacture of an agent for enhancing gastrointestinal activity.

(23) Use of ornithine or a salt thereof for the manufacture of an agent for preventing or ameliorating a symptom caused by reduced gastrointestinal activity.

(24) The use according to (23), wherein the symptom caused by reduced gastrointestinal activity is at least one symptom selected from indigestion, constipation, cachexia, anorexia nervosa, functional dyspepsia, and wasting disease.

(25) Ornithine or a salt thereof for use in secretion of ghrelin.

(26) Ornithine or a salt thereof for use in enhancement of eating activity.

(27) Ornithine or a salt thereof for use in prevention or amelioration of a symptom caused by reduced eating activity.

(28) The ornithine or a salt thereof according to (27), wherein the symptom caused by reduced eating activity is impairment in eating which does not involve organic injury in the gastrointestinal tract.

(29) The ornithine or a salt thereof according to (28), wherein the impairment in eating which does not involve organic injury in the gastrointestinal tract is at least one symptom selected from (impairment in eating due to functional dyspepsia, and impairment in eating due to age-related gastrointestinal malfunction.

(30) Ornithine or a salt thereof for use in enhancement of gastrointestinal activity.

(31) Ornithine or a salt thereof for use in prevention or amelioration of a symptom caused by reduced gastrointestinal activity.

(32) The ornithine or a salt thereof according to (31), wherein the symptom caused by reduced gastrointestinal activity is at least one symptom selected from indigestion, constipation, cachexia, anorexia nervosa, functional dyspepsia, and wasting disease.

Effects of the Invention

The present invention can provide an agent for enhancing eating activity and/or gastrointestinal activity which is safe and effective, and comprises ornithine or a salt thereof as an active ingredient.

Brief Description of Drawings

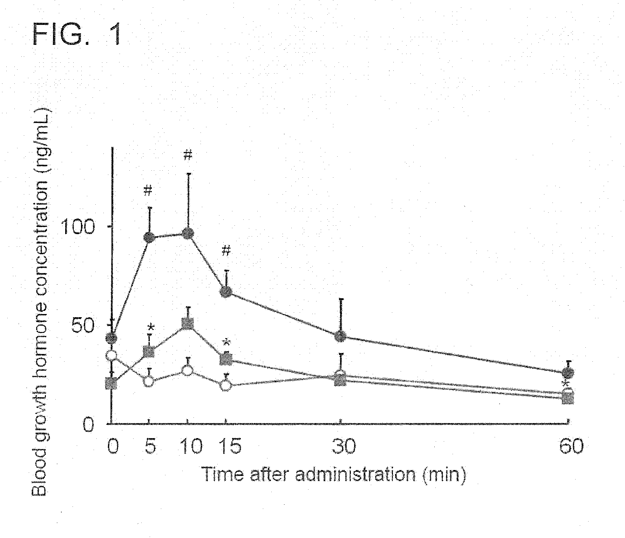

FIG. 1 shows enhancement of the ghrelin signaling system by which ornithine elicits the eating activity or gastrointestinal activity enhancing effect according to the present invention. In the figure, white circle, black circle, and black square represent the time-dependent changes of blood growth hormone for saline-administered control group (n = 5), ornithine hydrochloride-administered group (5 g/kg, n = 5), and ghrelin antagonist D-Lys3-GHRP-6 (0.8 mg/kg, i.v., n = 7) and ornithine hydrochloride-administered group (5 g/kg, n = 7), respectively. The symbol # in the figure means a statistically significant difference (P < 0.05) when comparing ornithine hydrochloride-administered group with saline-administered group, and the symbol * in the figure means a statistically significant difference (P < 0.05) when comparing ghrelin antagonist D-Lys3-GHRP-6 and ornithine hydrochloride-administered group with ornithine hydrochloride-administered group. Statistical processing was performed by using ANOVA and Fisher's test.

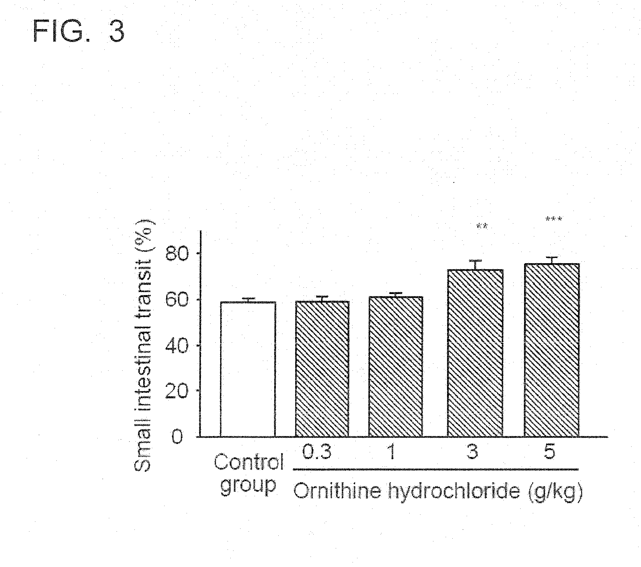

FIG. 2 represents the effect of intraduodenal administration of ornithine hydrochloride on blood ghrelin concentration (mean $\pm$ standard error; n = 4), showing that blood ghrelin concentration tends to increase due to ornithine hydrochloride administration.

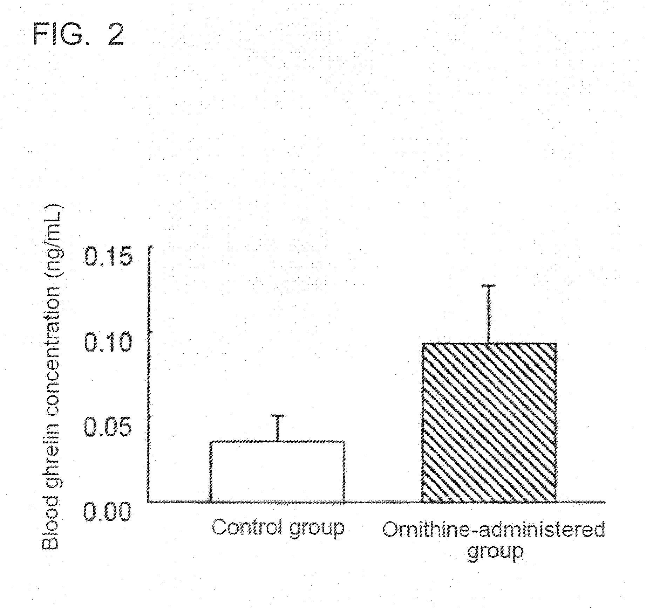

FIG. 3 shows the effect of ornithine on small intestinal transit (mean $\pm$ standard error; n = 4). The symbol ** in the figure means a statistically significant difference (P < 0.01) when comparing ornithine hydrochloride-administered group (3 g/kg) with control group, and the symbol *** in the figure means a statistically significant difference (P < 0.001) when comparing ornithine hydrochloride-administered group (5 g/kg) with control group. Statistical processing was performed by using ANOVA and Fisher's test.

Detailed Description of the Invention

Ornithine used in the present invention includes L-ornithine or D-ornithine, and preferably L-ornithine.

Ornithine used in the present invention may be obtained by any process. L-ornithine can be obtained, for example, by chemical synthesis method [Coll. Czechoslov. Chem. Commun., 24, 1993 (1959)], fermentation method (JP-A-1978-24096, JP-A-1986-119194), and the like. L-Ornithine and D-ornithine can also be purchased from Sigma-Aldrich, and the like.

Examples of the ornithine salt include an acid addition salt, a metal salt, an ammonium salt, an organic amine addition salt, an amino acid addition salt, and the like.

Examples of the acid addition salt include an inorganic acid salt, such as hydrochloride, sulfate, nitrate, or phosphate; and an organic acid salt, such as acetate, maleate, fumarate, citrate, malate, lactate, $\alpha$-ketoglutarate, gluconate, or caprylate.

Examples of the metal salt include an alkali metal salt, such as a sodium salt or a potassium salt; an alkali-earth metal salt, such as a magnesium salt or a calcium salt; an aluminum salt; and a zinc salt, and the like.

Examples of the ammonium salt include an ammonium salt, a tetramethylammonium salt, and the like.

Examples of the organic amine addition salt include a morpholine salt, a piperidine salt, and the like.

Examples of the amino acid addition salt include salts of glycine, phenylalanine, lysine, aspartic acid, glutamic acid, and the like.

Among the above salts of ornithine, hydrochloride or aspartate may preferably be used. However, another salt, or two or more combinations of the above salts may be optionally used.

As the agent for enhancing eating activity and/or gastrointestinal activity of the present invention, ornithine or a salt thereof can be administered as such. However, it is normally preferred to provide the agent as various kinds of preparations.

The preparation may be produced by mixing the active ingredient with one or more pharmaceutically acceptable carriers, and by using any method well known in the technical field of pharmaceuticals. The preparation may further comprise other active ingredients for any other treatment.

The preparation can be produced by using an additive, such as an excipient, a binder, a disintegrant, a lubricant, a dispersant, a suspension, an emulsifier, a diluent, a buffer, an antioxidizing agent, or a bacteria inhibitor.

The dosage form may be an oral form, such as a tablet, a powder, a granule, a pill, a suspension, an emulsion, an infusion/decoction, a capsule, a syrup, a liquid, an elixir, an extract, a tincture, or a fluidextract; or a parenteral form, such as an injection, a drop, a cream, or a suppository. Preferred is an oral form.

For example, when the dosage form suitable for oral administration is a tablet, a powder, or a granule, these can be prepared by adding a sugar (lactose, glucose, sucrose, mannitol, sorbitol, or the like); starch (potato, wheat, corn, or the like); an inorganic substance (calcium carbonate, calcium sulfate, sodium hydrogencarbonate, sodium chloride, or the like); an excipient, such as crystalline cellulose, or a plant powder (licorice powder, gentian powder, or the like); a disintegrant ,such as starch, agar, gelatin powder, crystalline cellulose, carmellose sodium, carmellose calcium, calcium carbonate, sodium hydrogencarbonate, or sodium alginate; a lubricant ,such as magnesium stearate, talc, hydrogenated vegetable oil, Macrogol, or silicone oil; a binder, such as polyvinyl alcohol, hydroxypropyl cellulose, methyl cellulose, ethyl cellulose, carmellose, gelatin, or starch paste; a surfactant, such as fatty acid ester; a plasticizer, such as glycerin; and the like.

When the dosage form suitable for oral administration is a liquid preparation, such as a syrup, the preparation can be prepared by adding water, a sugar (sucrose, sorbitol, fructose, or the like), glycol (polyethylene glycol, propylene glycol, or the like), an oil (sesame oil, olive oil, soybean oil, or the like), an antiseptic (p-hydroxybenzoate, or the like), a paraoxybenzoic acid derivative (methyl paraoxybenzoate, or the like), a preservative (sodium benzoate, or the like), a flavor (strawberry flavor, peppermint, or the like), and the like.

When the dosage form suitable for parenteral administration is an injection, the preparation comprises a sterilized aqueous preparation which comprises ornithine or a salt thereof, and which is preferably isotonic to the recipient's blood. For example, a solution for injection can be prepared by using a carrier comprising a salt solution, a glucose solution, or a mixture of a salt solution and a glucose solution, or the like.

The preparation suitable for oral administration may also contain an additive generally used in foods and drinks, such as a sweetener, a color, a preservative, a thickening stabilizer, an antioxidant, a color former, a bleaching agent, an anti-fungal agent, a gum base, a bittering agent, an enzyme, a brightening agent, an acidulant, a flavor enhancer, an emulsifier, a toughening agent, a production agent, a flavor, or a spice extract.

It is desirable to administer the preparation by the route that is the most effective for eating activity and/or gastrointestinal activity. Examples of the administration route include oral administration; parenteral administration, such as intravenous, intraperitoneal, or subcutaneous administration; and the like. Preferred is oral administration.

The concentration of the ornithine or a salt thereof in the agent for enhancing eating activity and/or gastrointestinal activity of the present invention is appropriately determined according to the type of preparation, the effect expected from the preparation administration, and the like. For example, in the case of an oral form, the concentration of ornithine or a salt thereof is normally 0.1 to 100 % by weight, preferably 0.5 to 80 % by weight, particularly preferably 1 to 70 % by weight.

The dose and the administration frequency of the agent for enhancing eating activity and/or gastrointestinal activity of the present invention depend on the dosage form, the age and the body weight of patients, and the nature or seriousness of the symptoms in need of treatment. Normally, the agent is given in a daily dose of 50 mg to 30 g, preferably 100 mg to 10 g, particularly preferably 200 mg to 3 g for adults in terms of an ornithine or a salt thereof, once to several times a day.

The agent for enhancing eating activity of the present invention can be used for an effect expected from enhancing eating activity.

The agent for enhancing gastrointestinal activity of the present invention can be used for an effect expected from enhancing gastrointestinal activity.

Further, the agent for eating activity of the present invention can be used for preventing or ameliorating a symptom caused by reduced eating activity. Examples of a symptom caused by reduced eating activity include impairment in eating caused by a symptom which is associated with impairment in eating, and which does not involve organic injury in the gastrointestinal tract (impairment in eating due to functional dyspepsia, impairment in eating due to age-related gastrointestinal malfunction, or the like) and the like. An individual presenting the symptom can be relieved from the symptom by administering the agent for enhancing eating activity of the present invention.

Further, the agent for enhancing gastrointestinal activity of the present invention can be used for preventing or ameliorating a symptom caused by reduced gastrointestinal activity. Examples of a symptom caused by reduced gastrointestinal activity include indigestion, constipation, cachexia, anorexia nervosa, functional dyspepsia, wasting disease, and the like. An individual presenting the symptom can be relieved from the symptom by administering the agent for enhancing gastrointestinal activity of the present invention.

Further, in the present invention, ornithine or a salt thereof can be used for the manufacture of the agent for enhancing eating activity and/or gastrointestinal activity.

Furthermore, the present invention includes a method for enhancing eating activity and/or gastrointestinal activity. The method of the present invention comprises the step of administering ornithine or a salt thereof to a subject in need of enhancing the eating activity and/or gastrointestinal activity in amounts sufficient for enhancing the eating activity and/or gastrointestinal activity of the subject.

The following describes test examples concerning the ghrelin secretion promoting effect that leads to the enhancement of eating activity and/or gastrointestinal activity by ornithine, as well as concerning the enhancement of gastrointestinal activity by ornithine.

Test Example 1

Changes in blood ghrelin concentration were examined according to the following testing method.

<Testing Method>

Male, 8 to 9-week Wistar rats were used.

Ornithine hydrochloride or saline as a control was administered to the duodenum, and blood growth hormone (GH) was measured over time. The results are presented in FIG. 1. The blood ghrelin concentration at 90 minutes after ornithine administration is shown in FIG. 2.

<Results>

As shown in FIG. 1, the ornithine hydrochloride-administered group had increased blood GH concentration levels compared to the control group. The GH concentration increasing effect of ornithine was inhibited by ghrelin antagonist D-Lys3-GHRP-6 (0.8 mg/kg, i.v.). The test was conducted under the condition where the antagonist alone does not show the effect. The result thus showed that the GH secretion promoting effect of ornithine is mediated by ghrelin secretion. Indeed, as shown in FIG. 2, the blood ghrelin concentration had the tendency to increase by ornithine administration. Taken together, the ghrelin secretion promoting effect of ornithine was confirmed.

Because it is known that ghrelin has the effect on enhancing eating activity (FASEB J. 2004, 18, 439-456), it is believed that ornithine or a salt thereof is useful as an agent for enhancing eating activity from the above-mentioned results.

Test Example 2

Gastrointestinal motility function was examined through small intestinal transit measurements according to the following testing method.

<Testing Method>

Male, 5 to 7-week ddY mice were used. The mice were fasted for 18 hours prior to the testing, and each animal (n = 4) was orally administered with 0.3, 1, 3, or 5 g/kg (body weight) of ornithine hydrochloride, or saline as a control. After 30 minutes, the mice were orally administered with a dyed test meal (Evans Blue 5%, carboxymethyl cellulose 1%). The animals were killed by cervical dislocation after 5 minutes, and immediately cut open in the stomach to remove the whole small intestine. For evaluation of the gastrointestinal motility, the full length of the small intestine, and the length from the pylorus to the farthest point marked by the movement of Evans Blue were measured. Evans Blue mobility was calculated according to the following equation, and used as an index.

$$\text{Mobility (\%)} = (\text{the length from the pylorus to the farthest point marked by the movement of Evans Blue} \,/\, \text{the full length of small intestine}) \times 100$$

<Results>

As shown in FIG. 3, the ornithine hydrochloride-administered group had a significantly increased level of small intestinal transit compared to the control group, demonstrating that the ornithine had the promoting effect of the gastrointestinal motility ($P < 0.01$ for the ornithine hydrochloride administration of 3 g/kg (body weight), and $P < 0.001$ for the ornithine hydrochloride administration of 5 g/kg (body weight)).

Examples of the present invention are described below.

Example 1

Production of Tablet Comprising Ornithine

Ornithine hydrochloride (136.2 kg; L-ornithine hydrochloride, Kyowa Hakko Bio), microcrystalline cellulose (36.0 kg; Avicel FD101, Asahi Kasei Chemicals Corporation), sucrose fatty acid ester (6.6 kg; DK ester F-20W, Dai-Ichi Kogyo Seiyaku Co., Ltd.), calcium phosphate (1.2 kg; tricalcium phosphate, Taihei Chemical Industrial Co., Ltd.), and β-cyclodextrin (20.0 kg; Cerdex B-100, Nihon Shokuhin Kako Co., Ltd.) were mixed by using a conical blender (CB-1200 blender; Nihon Kansouki Co., Ltd.). The resulting mixture was compression-molded under the compression molding pressure of 10 kN with a rotary compression molding machine (VIRGO524SS1AY; Kikusui Seisakusho) to produce tablets (diameter 8 mm; 250 mg).

Example 2

Production of Enteric Capsule Comprising Ornithine

The mixture (20 kg) prepared in Example 1, and silicon dioxide (0.2 kg) were mixed and stirred. The resulting mixture was charged into a capsule filling machine and filled in 20,000 gelatin hard capsules (size 2). Surface of the resulting hard capsules were then coated with zein solution using Hi-Coater HCT-48 (Freund) to produce 20,000 enteric capsules comprising ornithine hydrochloride.

Example 3

Production of Enteric Tablet Comprising Ornithine

Surface of the tablets prepared in Example 1 were coated with shellac solution using Hi-Coater HCT-48 (Freund) to produce enteric tablets.

Example 4

Production of Drink Comprising Ornithine

Ornithine hydrochloride (1.28 kg; L-ornithine hydrochloride, Kyowa Hakko Bio), erythritol (3 kg; Nikken Chemical Laboratory), citric acid (0.05 kg; Kyowa Hi Foods), artificial sweetener (3 g), and flavor (0.06 kg) were stirred and dissolved in 50 L of water at a liquid temperature of 70°C. After being adjusted to pH 3.3 with citric acid, the solution was sterilized with a plate sterilizer, and charged into a bottle, followed by sterilization with a pasteurizer, to produce a drink.

Industrial Applicability

The present invention can provide an agent for enhancing eating activity and/or gastrointestinal activity which is safe and effective, and comprises ornithine or a salt thereof as an active ingredient.

## Claims

1. An agent for secreting ghrelin, comprising ornithine or a salt thereof as an active ingredient.

2. An agent for enhancing eating activity, comprising ornithine or a salt thereof as an active ingredient.

3. An agent for preventing or ameliorating a symptom caused by reduced eating activity, comprising ornithine or a salt thereof as an active ingredient.

4. The agent for prevention or amelioration according to claim 3, wherein the symptom caused by reduced eating activity is impairment in eating which does not involve organic injury in the gastrointestinal tract.

5. The agent for prevention or amelioration according to claim 4, wherein the impairment in eating which does not involve organic injury in the gastrointestinal tract is at least one symptom selected from impairment in eating due to functional dyspepsia, and impairment in eating due to age-related gastrointestinal malfunction.

6. An agent for enhancing gastrointestinal activity, comprising ornithine or a salt thereof as an active ingredient.

7. An agent for preventing or ameliorating a symptom caused by reduced gastrointestinal activity, comprising ornithine or a salt thereof as an active ingredient.

8. The agent for prevention or amelioration according to claim 7, wherein the symptom caused by reduced gastrointestinal activity is at least one symptom selected from indigestion, constipation, cachexia, anorexia nervosa, functional dyspepsia, and wasting disease.

9. A method for secreting ghrelin comprising the step of administering an effective amount of ornithine or a salt thereof.

10. A method for enhancing eating activity comprising the step of administering an effective amount of ornithine or a salt thereof.

11. A method for preventing or ameliorating a symptom caused by reduced eating activity, comprising the step of administering an effective amount of ornithine or a salt thereof.

12. The method for prevention or amelioration according to claim 11, wherein the symptom caused by reduced eating activity is impairment in eating which does not involve organic injury in the gastrointestinal tract.

13. The method for prevention or amelioration according to claim 12, wherein the impairment in eating which does not involve organic injury in the gastrointestinal tract is at least one symptom selected from impairment in eating due to functional dyspepsia, and impairment in eating due to age-related gastrointestinal malfunction.

14. A method for enhancing gastrointestinal activity, comprising the step of administering an effective amount of ornithine or a salt thereof.

15. A method for preventing or ameliorating a symptom caused by reduced gastrointestinal activity, comprising the step of administering an effective amount of ornithine or a salt thereof.

16. The method for prevention or amelioration according to claim 15, wherein the symptom caused by reduced gastrointestinal activity is at least one symptom selected from indigestion, constipation, cachexia, anorexia nervosa, functional dyspepsia, and wasting disease.

17. Use of ornithine or a salt thereof for the manufacture of an agent for secreting ghrelin.

18. Use of ornithine or a salt thereof for the manufacture of an agent for enhancing eating activity.

19. Use of ornithine or a salt thereof for the manufacture of an agent for preventing or ameliorating a symptom caused by reduced eating activity.

20. The use according to claim 19, wherein the symptom caused by reduced eating activity is impairment in eating which does not involve organic injury in the gastrointestinal tract.

21. The use according to claim 20, wherein the impairment in eating which does not involve organic injury in the gastrointestinal tract is at least one symptom selected from impairment in eating due to functional dyspepsia, and impairment in eating due to age-related gastrointestinal malfunction.

22. Use of ornithine or a salt thereof for the manufacture of an agent for enhancing gastrointestinal activity.

23. Use of ornithine or a salt thereof for the manufacture of an agent for preventing or ameliorating a symptom caused by reduced gastrointestinal activity.

24. The use according to claim 23, wherein the symptom caused by reduced gastrointestinal activity is at least one symptom selected from indigestion, constipation, cachexia, anorexia nervosa, functional dyspepsia, and wasting disease.

25. Ornithine or a salt thereof for use in secretion of ghrelin.

26. Ornithine or a salt thereof for use in enhancement of eating activity.

27. Ornithine or a salt thereof for use in prevention or amelioration of a symptom caused by reduced eating activity.

28. The ornithine or a salt thereof according to claim 27, wherein the symptom caused by reduced eating activity is impairment in eating which does not involve organic injury in the gastrointestinal tract.

29. The ornithine or a salt thereof according to claim 28, wherein the impairment in eating which does not involve organic injury in the gastrointestinal tract is at least one symptom selected from impairment in eating due to functional dyspepsia, and impairment in eating due to age-related gastrointestinal malfunction.

**30.** Ornithine or a salt thereof for use in enhancement of gastrointestinal activity.

**31.** Ornithine or a salt thereof for use in prevention or amelioration of a symptom caused by reduced gastrointestinal activity.

**32.** The ornithine or a salt thereof according to claim 31, wherein the symptom caused by reduced gastrointestinal activity is at least one symptom selected from indigestion, constipation, cachexia, anorexia nervosa, functional dyspepsia, and wasting disease.

# FIG. 1

FIG. 2

FIG. 3

## INTERNATIONAL SEARCH REPORT

| International application No. |
|---|
| PCT/JP2013/055617 |

### A.   CLASSIFICATION OF SUBJECT MATTER

*A61K31/198*(2006.01)i, *A61P1/00*(2006.01)i, *A61P1/10*(2006.01)i, *A61P1/14* (2006.01)i, *A23L1/305*(2006.01)n, *A23L2/52*(2006.01)n

According to International Patent Classification (IPC) or to both national classification and IPC

### B.   FIELDS SEARCHED

Minimum documentation searched (classification system followed by classification symbols)
A61K31/198, A61P1/00, A61P1/10, A61P1/14, A23L1/305, A23L2/52

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAPLUS/REGISTRY/BIOSIS/MEDLINE/WPIDS/EMBASE(STN)

### C.   DOCUMENTS CONSIDERED TO BE RELEVANT

| Category* | Citation of document, with indication, where appropriate, of the relevant passages | Relevant to claim No. |
|---|---|---|
| X<br>A | JP 2002-541077 A   (Societe de Conseils de Recherches et d'Applications Scientifiques (S.C.R.A.S.)),<br>03 December 2002 (03.12.2002),<br>claims 1, 8 to 9, 13 to 20; paragraphs [0002], [0012] | 2-8,18-32<br>1,17 |
| X<br>A | WO 2006/030980 A1   (Ajinomoto Co., Inc.),<br>23 March 2006 (23.03.2006),<br>claims 1, 4 to 5; page 14, lines 9 to 22 | 2-8,18-32<br>1,17 |
| A | JP 2005-82489 A   (Kyoto University),<br>31 March 2005 (31.03.2005),<br>entire text | 1-32 |
| A | A.Inui, et al., The FASAB Journal, 2004, 18, pp439-456, entire text | 1-32 |

☐   Further documents are listed in the continuation of Box C.          ☒   See patent family annex.

| | |
|---|---|
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    27 March, 2013 (27.03.13) | Date of mailing of the international search report<br>    09 April, 2013 (09.04.13) |
|---|---|
| Name and mailing address of the ISA/<br>    Japanese Patent Office | Authorized officer |
| Facsimile No. | Telephone No. |

Form PCT/ISA/210 (second sheet) (July 2009)

<table>
<tr><td colspan="2"><strong>INTERNATIONAL SEARCH REPORT</strong><br>Information on patent family members</td><td>International application No.<br>PCT/JP2013/055617</td></tr>
</table>

| | | |
|---|---|---|
| JP 2002-541077 A | 2002.12.3 | EP 1169005 A<br>WO 2000/059448 A2<br>FR 2791571 A<br>FR 2791571 A1<br>AU 3663700 A<br>NO 20014770 A<br>CA 2365500 A<br>AR 23219 A |
| WO 2006/030980 A1 | 2006.3.23 | JP 4124260 B<br>JP 2008-115185 A<br>US 2007/0218112 A1<br>US 2009/0291910 A1<br>EP 1806134 A1<br>EP 2103306 A2<br>EP 2305241 A1<br>CA 2580692 A<br>KR 10-2007-0053352 A<br>CN 101052390 A<br>BRA PI0515413<br>KR 10-2009-0086450 A<br>RU 2009110912 A<br>AU 2005283297 A<br>RU 2407524 C<br>MX 2007003196 A<br>KR 10-1086628 B<br>CN 102379865 A<br>CN 102379891 A<br>RU 2010136914 A<br>ES 2384294 T<br>ES 2388609 T |
| 2005-82489 A | 2005.3.31 | (Family: none) |

Form PCT/ISA/210 (patent family annex) (July 2009)

**INTERNATIONAL SEARCH REPORT**

| International application No. |
|---|
| PCT/JP2013/055617 |

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 9-16
   because they relate to subject matter not required to be searched by this Authority, namely:
   Claims 9-16 pertain to a method for treatment of the human body by therapy and thus relate to a subject matter on which this International Searching Authority is not required to carry out a search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007040244 A
- JP 2006342148 A
- JP 2007031375 A
- JP 2007119348 A
- JP 2008214232 A
- JP 53024096 A
- JP 61119194 A

**Non-patent literature cited in the description**

- *Coll. Czechoslov. Chem. Commun.,* 1959, vol. 24, 1993
- *FASEB J.,* 2004, vol. 18, 439-456